# EUROPEAN PATENT APPLICATION

(11) **EP 3 311 910 A1**
(43) Date of publication of application: **25.04.2018**
(21) Application number: 16811500.4
(22) Date of filing: 07.06.2016
(51) Int. Cl.: B01J 4/00, A61L 2/20, A61L 101/02, A61L 101/22, A61L 101/44

(54) **DEVICE TO WHICH GAS IS SUPPLIED, GAS SUPPLY SYSTEM, AND CONTROL METHOD FOR GAS SUPPLY SYSTEM**

(30) Priority: 19.06.2015 JP 2015123913
(71) Applicant: Sinfonia Technology Co., Ltd., Tokyo 105-8564 (JP)
(72) Inventor: YAMADA, Takayuki, Tokyo 105-8564 (JP); TAKEUCHI, Haruki, Tokyo 105-8564 (JP)
(74) Representative: Cabinet Beau de Loménie
(86) International application number: PCT/JP2016/066911
(87) International publication number: WO 2016/204025

(57) **Abstract**

Even when power supply to a gas supply target device is stopped and then resumed, the state of gas supply to a chamber is grasped and a process is performed in accordance with the state.

The gas supply target device includes a retaining unit (12) which is able to selectively take one of three states A to C each indicating a state of gas supply to the chamber 10a and is able to maintain, when power supply to the gas supply target device is stopped, one of the states at stop of the power supply. When power supply to the gas supply target device is stopped and then resumed, in which one of the states A to C the retaining unit (12) is set is determined, and a process is performed in accordance with the state of the gas supply to the chamber (10a).

## Description

[Technical Field] The present invention relates to a gas supply target device including a chamber to which gas such as nitrogen gas and hydrogen peroxide gas is supplied, a gas supply system including the gas supply target device and a gas generator, and a method of controlling the gas supply system.

[Background Art] There is a known technology by which, in a step of processing a substrate such as a semiconductor wafer and a liquid crystal display panel, the substrate is housed in a chamber of a gas supply target device and inert gas such as nitrogen gas is supplied to the chamber, with the result that pollutant is removed from the chamber (and the substrate housed in the chamber) (see Patent Literature 1). In addition to this, there is a known technology by which, in a step of culturing and testing cells, a step of manufacturing medical appliances, and the like, gas with a sterilizing effect, such as hydrogen peroxide gas, is supplied to a chamber of a gas supply target device to sterilize the chamber (and a sterilization target when it is housed in the chamber) (see Patent Literature 2).

### [Citation List][Patent Literatures]

Patent Literature 1: WO2010/137556
Patent Literature 2: Japanese Unexamined Patent Publication No. 2013-144159

### [Summary of Invention]

### [Technical Problem]

When one of the above-described technologies is implemented, power supply to the gas supply target device may be stopped on account of a power failure or the like, and the power supply may be resumed thereafter. In such a case, if the state of gas supply to the chamber (e.g., before the start of the gas supply, during the gas supply, and after the completion of the gas supply) is not grasped, the following problems may occur. For example, because the state of the gas supply to the chamber is unknown, the work efficiency is low if aeration (which is a process of circulating air between the chamber and the outside by supplying the air to the chamber and exhausting the air from the chamber in order to lower the gas concentration in the chamber) is simply conducted. Furthermore, for example, when a door of the chamber is opened even if the state of the gas supply to the chamber is unknown, the gas leaks out from the chamber. When the gas is harmful to the human body, operating personnel may be exposed to risk.

An object of the present invention is to provide a gas supply target device, a gas supply system, and a method of controlling the gas supply system, which make it possible to grasp the state of the gas supply to a chamber and to perform a process in accordance with the state even when power supply to the gas supply target device is stopped and then resumed.

### [Solution to Problem]

The first aspect of the present invention provides a gas supply target device which includes a chamber to which gas is supplied, the gas supply target device further including a retaining unit which is able to selectively take one of a plurality of states each indicating a state of gas supply to the chamber and is able to maintain, when power supply to the gas supply target device is stopped, one of the states which is set at stop of the power supply.

The second aspect of the present invention provides a gas supply system which comprises: a gas supply target device including a chamber to which gas is supplied; and a gas generator including a gas generating unit configured to generate the gas, the gas generated by the gas generating unit being supplied to the chamber, the gas supply target device further including a retaining unit which is able to selectively take one of a plurality of states each indicating a state of gas supply to the chamber and is able to maintain, when power supply to the gas supply target device is stopped, one of the states at stop of the power supply, and the gas supply system further comprising: a determination unit configured to determine in which one of the states the retaining unit is; and a processing unit configured to perform a process in accordance with determination by the determination unit.

The third aspect of the present invention provides a method of controlling a gas supply system which comprises a gas supply target device including a chamber to which gas is supplied and a gas generator including a gas generating unit configured to generate the gas, the gas supply system being configured to supply the gas generated by the gas generator to the chamber, the gas supply target device further including a retaining unit which is able to selectively take one of a plurality of states each indicating a state of gas supply to the chamber and is able to maintain, when power supply to the gas supply target device is stopped, one of the states at stop of the power supply, and the method comprising the step of: determining, by a determination unit of the gas supply system, in which one of the states the retaining unit is; and performing, by a processing unit of the gas supply system, a process in accordance with determination of the determination unit.

According to the first to the third aspects, when power supply to a gas supply target device is stopped and then resumed, the state of the retaining unit is determined and the state of the gas supply to a chamber is grasped, and a process is performed in accordance with the state.

According to the first to third aspects, the retaining unit may be configured so that an external device be able to determine, by using the retaining unit, which one of the states is set, both when the power supply to the gas supply target device is in process and when the power supply is stopped. According to this arrangement, both when power supply to the gas supply target device is in process and when the power supply is stopped, the external device is able to grasp the state of the retaining unit in the gas supply target device and the state of the gas supply to the chamber of the gas supply target device, and to perform a process suitable for these states.

According to the first to third aspects, when the power supply to the gas supply target device is in process, the retaining unit may be settable in one of the states by an external device. According to this arrangement, the gas supply target device is further suitably managed by the external device.

According to the first to third aspects, the retaining unit may include a latching relay. According to this arrangement, the state of the gas supply to the chamber is accurately grasped by electrically determining the state of the latching relay. It is therefore possible to perform a process suitable for the state.

According to the first to third aspects, the gas may have a sterilizing effect. The sterilizing effect indicates an effect to achieve complete elimination or removal of all kinds of proliferative microbiological organisms (mainly bacteria). Depending on the concentration, the gas having the sterilizing effect may be harmful for human body. According to the arrangement above, the advantage of the present invention is therefore significant in terms of the security of the operating personnel.

The gas supply system of the second aspect may comprise at least one gas supply target device each of which is identical with the gas supply target device, the gas generated by the gas generating unit being supplied to the chamber of one gas supply target device which is selected one by one from the gas supply target devices. According to this arrangement, when the gas is supplied to the chambers of the gas supply target devices one by one, it is possible to grasp the state of the gas supply to each gas supply target device and perform a process suitable for the state.

According to the first to third aspects, the retaining unit may selectively take one of three or more states. According to this arrangement, because the retaining unit is able to selectively take not one of only two states but one of three or more states, it is possible to grasp the state of the gas supply to the chamber in a detailed manner and to realize fine management of the gas supply target device.

According to the first to the third aspects, the retaining unit may include a plurality of elements, the state of each of which is switchable. According to this arrangement, the arrangement that the retaining unit is able to selectively take one of three or more states is relatively easily realized by combinations of the states of the elements.

### [Advantageous Effects of Invention]

According to the present invention, when power supply to a gas supply target device is stopped and then resumed, the state of the retaining unit is determined and the state of the gas supply to a chamber is grasped, and a process is performed in accordance with the state.

### [Brief Description of Drawings]

[FIG. 1] FIG. 1 is a schematic diagram showing a gas supply system of an embodiment of the present invention.
[FIG. 2] FIG. 2 is a block diagram showing an electric structure of the gas supply system of the embodiment of the present invention.
[FIG. 3] FIG. 3 shows the correlation between the states of gas supply (before the start of the gas supply, during the gas supply, and after the completion of the gas supply) to a chamber and the states of a retaining unit (state A, state B, and state C) in the embodiment of the present invention.
[FIG. 4] FIG. 4 is a flowchart of a method of controlling the gas supply system of the embodiment of the present invention.
[FIG. 5] FIG. 5 is a flowchart of a door lock control routine.

### [Description of Embodiments]

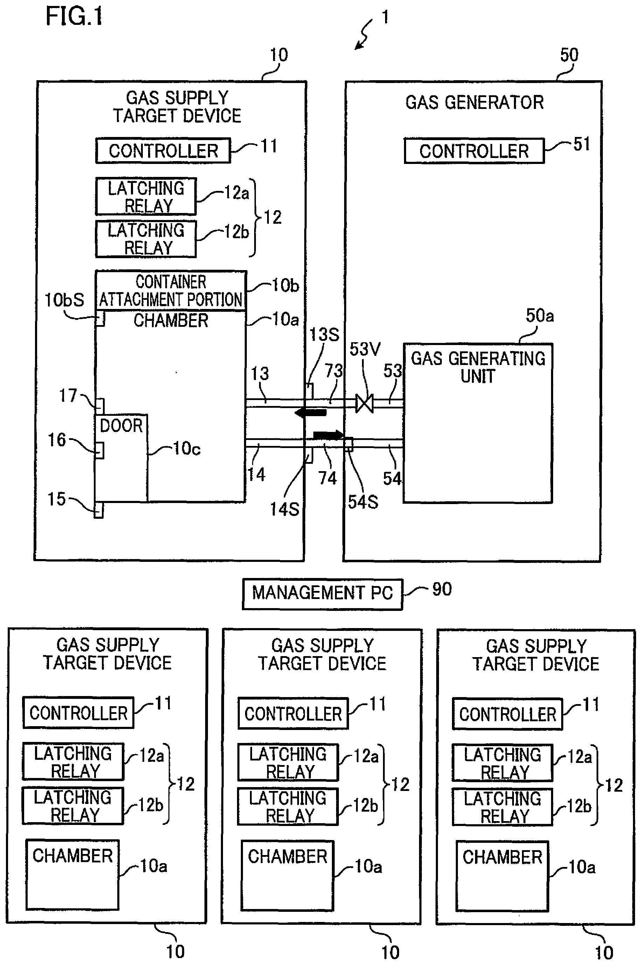

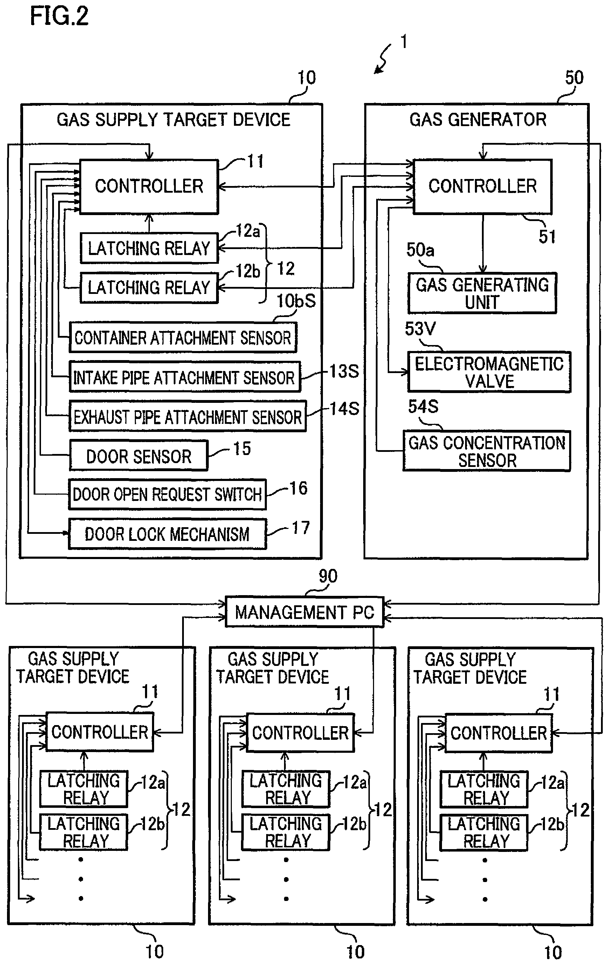

As shown in FIG. 1 and FIG. 2, a gas supply system 1 of an embodiment of the present invention includes a plurality of gas supply target devices 10, a gas generator 50, and a management PC (Personal Computer) 90 configured to centrally control the devices 10 and 50.

Each gas supply target device 10 includes members such as a controller 11, a retaining unit 12, and a chamber 10a. The retaining unit 12 includes two latching relays 12a and 12b. In particular, the gas supply target device 10 which is connected to the gas generator 50 in FIG. 1 and FIG. 2 further includes a container attachment portion 10b, a container attachment sensor 10bS, a door 10c, a pipe 13 constituting a passage for supplying gas to the chamber 10a, a pipe 14 constituting an exhaust passage from the chamber 10a, an intake pipe attachment sensor 13S, an exhaust pipe attachment sensor 14S, a door sensor 15, an door open request switch 16, and a door lock mechanism 17.

The gas generator 50 includes a controller 51, a gas generating unit 50a, a pipe 53 constituting a passage for supplying gas to the chamber 10a, a pipe 54 constituting an exhaust passage from the chamber 10a, an electromagnetic valve 53V, and a gas concentration sensor 54S.

The chamber 10a is a space to which gas is supplied.

The container attachment portion 10b is provided above the chamber 10a. To and from the container attachment portion 10b, a container (which is a container capable of storing plural cell culture dishes) is attachable and detachable. When a container is attached to the container attachment portion 10b, the internal space of the container communicates with the chamber 10a.

The container attachment sensor 10bS is provided in the vicinity of the container attachment portion 10b and is configured to send, to the controller 11, an ON signal when the container is attached to the container attachment portion 10b or an OFF signal when the container is not attached to the container attachment portion 10b. The door 10c is attached to a side wall which defines the chamber 10a, and is openable and closable by operating personnel. When the door 10c is open, the chamber 10a communicates with the outside space.

The door sensor 15 is provided in the vicinity of the door 10c and is configured to send, to the controller 11, an ON signal when the door 10c is closed or an OFF signal when the door 10c is open.

The door open request switch 16 is provided in the vicinity of the door 10c and is pressed by operating personnel when it is necessary to open the door 10c. The door open request switch 16 sends, to the controller 11, an ON signal when pressed or an OFF signal when not pressed.

The door lock mechanism 17 is a mechanism for prohibiting the door 10c from opening. This mechanism is formed of suitable mechanisms (e.g., a solenoid and a gear). Under the control of the controller 11, the door lock mechanism 17 selectively takes an ON state (in which the door 10c is prohibited from opening) or an OFF state (in which the door 10c is not prohibited from opening). For safety, the door lock mechanism 17 is maintained in the ON state (in which the door 10c is prohibited from opening) when power supply to the gas supply target device 10 is stopped.

The intake pipe attachment sensor 13S and the exhaust pipe attachment sensor 14S are provided in the vicinity of leading ends (which are opposite to the leading ends connected to the chamber 10a) of the pipes 13 and 14, respectively. To the controller 11, each of these sensors sends an ON signal when an intake pipe 73 or an exhaust pipe 74 is attached to the leading end, or sends an OFF signal when the intake pipe 73 or the exhaust pipe 74 is not attached to the leading end.

The intake pipe 73 connects the pipe 13 to the pipe 53. The exhaust pipe 74 connects the pipe 14 to the pipe 54.

The gas generating unit 50a generates gas under the control of the controller 51. The gas generated by the gas generating unit 50a is supplied to the chamber 10a via the pipes 53, 73, and 13.

The electromagnetic valve 53V is provided in the middle of the pipe 53. Under the control of the controller 51, the valve is able to selectively take an open state or a closed state. When the electromagnetic valve 53V is in the open state, the pipe 53 is able to communicate with the outside and gas is allowed to flow from the pipe 53 to the pipes 73 and 13. When the electromagnetic valve 53V is in the closed state, the communication between the pipe 53 and the outside is blocked and gas is not allowed to flow from the pipe 53 to the pipes 73 and 13.

The gas concentration sensor 54S is provided at a leading end (which is an end portion opposite to the end portion connected to the gas generating unit 50a) of the pipe 54. This sensor detects the concentration of the gas passing the leading end and sends a detection signal to the controller 51.

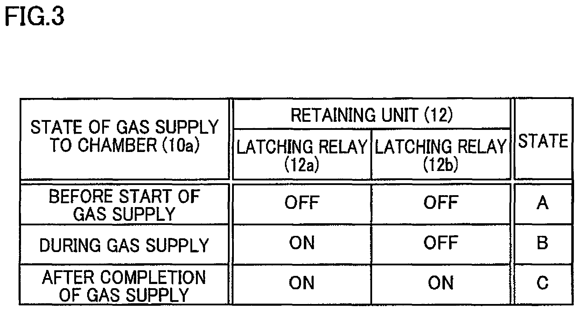

Each of the latching relays 12a and 12b is provided with a contact which is switched ON or OFF in accordance with an input signal sent thereto from the controller 51 of the gas generator 50, and maintains the ON state or the OFF state even if no input signal is further sent. As shown in FIG. 3, the retaining unit 12 arranged as above is able to selectively take one of the three states A to C corresponding to combinations of ON and OFF of the two latching relays 12a and 12b, and is able to retain the state at the stop of power supply when power supply to the gas supply target device 10 is stopped. Each of the states A to C indicates the state of gas supply to the chamber 10a. The state A is a state in which both of the latching relays 12a and 12b are switched OFF and indicates "before the start of the gas supply". The state B is a state in which the latching relay 12a is switched on whereas the latching relay 12b is switched off, and indicates "during the gas supply". The state C is a state in which both of the latching relays 12a and 12b are switched ON and indicates "after the completion of the gas supply".

The controller 51 of the gas generator 50 is able to switch ON or OFF each of the latching relays 12a and 12b, and is able to detect that each of the latching relays 12a and 12b is switched ON or OFF. (In other words, the controller is able to perform writing into each of the latching relays 12a and 12b and to read the ON or OFF state of each of the latching relays 12a and 12b.) The writing into and reading from each of the latching relays 12a and 12b by the controller 51 can be done both when power supply to the gas supply target device 10 is in process and when the power supply is stopped. To put it differently, the retaining unit 12 is configured so that an external device (which is the controller 51 of the gas generator 50 in the present embodiment) be able to determine, by using the retaining unit 12, which one of the states A to C is set, both when power supply to the gas supply target device 10 is in process and when the power supply is stopped. Furthermore, when power supply to the gas supply target device 10 is in process, the retaining unit 12 can be set in one of the states A to C not by that gas supply target device 10 but by an external device (which is the controller 51 of the gas generator 50 in the present embodiment).

In the meanwhile, the controller 11 of the gas supply target device 10 is able to detect that each of the latching relays 12a and 12b is switched ON or OFF but cannot switch each of the latching relays 12a and 12b ON or OFF.

The management PC 90 is connected to the controller 11 of each gas supply target device 10 and the controller 51 of the gas generator 50 to be able to communicate therewith. The management PC is able to send an instruction to each of the controllers 11 and 51 and to read information from each of the controllers 11 and 51. Furthermore, the management PC 90 stores information regarding each of the devices 10 and 50 (e.g., history of the gas supply).

In the present embodiment, the gas generated by the gas generating unit 50a and supplied to the chamber 10a is hydrogen peroxide gas which has a sterilizing effect. To put it differently, the gas supply system 1 of the present embodiment is configured to sterilize, by using hydrogen peroxide gas, the chamber 10a (and a sterilization target when the sterilization target is housed in the chamber 10a) . (In the gas supply target device 10 connected to the gas generator 50 via the pipes 73 and 74 shown in FIG. 1 and FIG. 2, the sterilization target is cell culture dishes stored in the container.) A cell and a culture solution are put in each of the sterilized cell culture dishes for the purpose of culture of the cell and a subsequent test.

Gas supply target devices 10 which are not connected to the gas generator 50 in FIG. 1 and FIG. 2 are a culture apparatus or an inspection apparatus for cells, a device constituting a passage through which a cell culture dish is moved to the culture apparatus or the inspection apparatus, and the like. These gas supply target devices are not connected to the gas generator 50 at this stage but are connected to the gas generator 50 one by one via the pipes 73 and 74, and gas is supplied to the chamber 10a. In other words, the gas supply system 1 of the present embodiment is arranged to supply the gas generated by the gas generating unit 50a to the chamber 10a of one gas supply target device 10 which is selected one by one from the gas supply target devices 10.

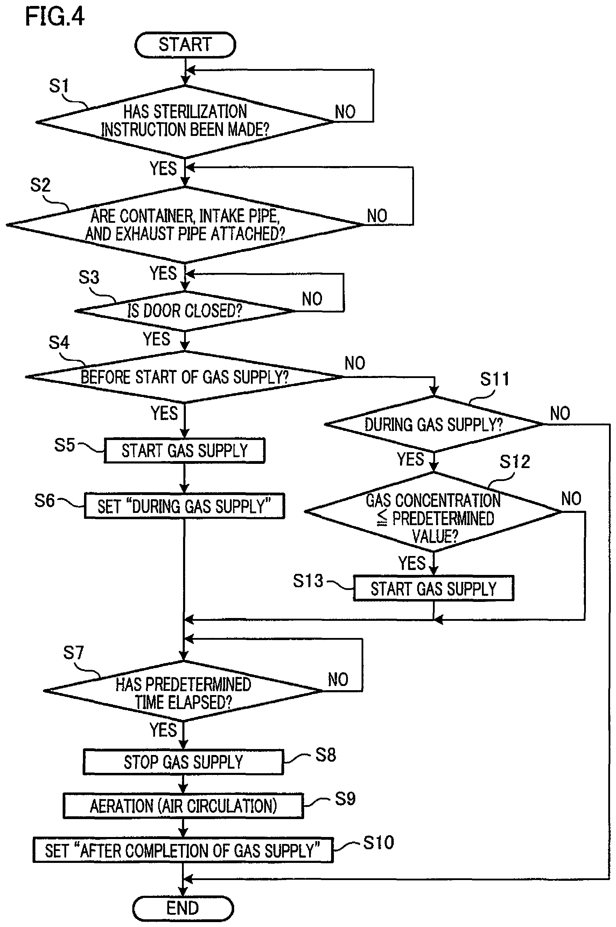

Now, a method of controlling the gas supply system 1 of the embodiment of the present invention will be described with reference to FIG. 4. The control described below is executed by the management PC 90, the controller 11 of the gas supply target device 10 connected to the gas generator 50, and the controller 51 of the gas generator 50.

As the gas supply target device 10 connected to the gas generator 50, the gas generator 50, and the management PC 90 are activated (including activation in a case where power supply to the devices 10 and 50 and the management PC 90 is stopped due to a power failure or the like and then the power supply is resumed and activation in a case where the gas supply to a gas supply target device 10 is stopped not by a power failure but by detachment of the intake pipe 73 and/or the exhaust pipe 74 and then the gas supply target device 10 is connected again to the gas generator 50), to begin with, the management PC 90 determines whether a sterilization instruction has been made to the gas supply target device 10 connected to the gas generator 50 (S1). The sterilization instruction is, for example, input by operating personnel via input means provided at the management PC 90.

When no sterilization instruction has been made (NO in S1), the management PC 90 repeats the step S1.

When the sterilization instruction has been made (YES in S1), the controller 11 of that gas supply target device 10 determines whether the container, the intake pipe 73, and the exhaust pipe 74 are attached, based on signals from sensors lObS, 13S, and 14S (S2).

When at least one of the container, the intake pipe 73, and the exhaust pipe 74 is not attached (NO in S2), the controller 11 repeats the step S2.

When all of the container, the intake pipe 73, and the exhaust pipe 74 are attached (YES in S2), the controller 11 determines whether the door 10c is closed, based on a signal from the door sensor 15 (S3) .

When the door 10c is not closed (NO in S3), the controller 11 repeats the step S3.

When the door 10c is closed (YES in S3), the controller 11 determines whether the retaining unit 12 is set in the state A which indicates "before the start of the gas supply" (S4).

"Before the start of the gas supply" or "after the completion of the gas supply", the gas concentration in the chamber 10a is relatively low and lower than a constant value (which is, for example, concentration harmful for human body). "During the gas supply", the gas concentration in the chamber 10a is relatively high and is higher than the constant value. "After the gas supply", it would be unnecessary to further supply the gas to the chamber 10a because the sterilization of the chamber 10a has already been completed.

When the retaining unit 12 is in the state A indicating "before the start of the gas supply" (YES in S4), the controller 51 of the gas generator 50 switches the electromagnetic valve 53V to the open state and drives the gas generating unit 50a so as to start the gas supply to the chamber 10a (S5). During the gas supply to the chamber 10a, exhaust from the chamber 10a via the pipes 14, 74, and 54 is executed along with the gas supply via the pipes 53, 73, and 13. The exhaust gas is collected by the gas generating unit 50a.

After S5, the controller 51 sends an input signal to the latching relays 12a and 12b to set the retaining unit 12 in the state B (indicating "during the gas supply") (S6).

After S6, the controller 51 determines whether a predetermined time (i.e., a time until the gas concentration in the chamber 10a reaches a predetermined value) has elapsed from the activation of the gas generating unit 50a in S5 (S7).

When the predetermined time has not elapsed (NO in S7), the controller 51 repeats the step S6.

When the predetermined time has elapsed (YES in S7), the controller 51 stops the gas generating unit 50a and switches the electromagnetic valve 53V to the closed state so as to stop the gas supply to the chamber 10a (S8).

After S8, the controller 51 performs, by using a catalyst or the like, aeration (which is a process of circulating air between the chamber 10a and the gas generating unit 50a by supplying the air to the chamber 10a and exhausting the air from the chamber 10a in order to lower the gas concentration in the chamber 10a) (S9). The controller 51 performs the aeration until the gas concentration becomes lower than the constant value, based on a detection signal from the gas concentration sensor 54S.

After S9, the controller 51 sends an input signal to the latching relays 12a and 12b to set the retaining unit 12 in the state C (indicating "after the completion of the gas supply") (S10). The control routine ends after S10.

When the retaining unit 12 is not in the state A indicating "before the start of the gas supply" (NO in S4), the controller 11 of the gas supply target device 10 determines whether the retaining unit 12 is in the state B indicating "during the gas supply" (S11).

When the retaining unit 12 is in the state B indicating "during the gas supply" (YES in S11), the controller 51 of the gas generator 50 determines whether the gas concentration is not higher than a predetermined value based on a detection signal from the gas concentration sensor 54S (S12).

When the gas concentration is equal to or lower than the predetermined value (YES in S12), the controller 51 switches the electromagnetic valve 53V to the open state and drives the gas generating unit 50a so as to start the supply of the gas to the chamber 10a in the same manner as in S5 (S13).

After S13 or when the gas concentration is higher than the predetermined value (NO in S12), the controller 51 proceeds to S7. The predetermined time in S7 in this case is determined based on the concentration detected in S12.

When the retaining unit 12 is not in the state B indicating "during the gas supply" (i.e., in the state C indicating "after the completion of the gas supply") (NO in S11), the control routine ends.

Because the gas supply is not unnecessarily done when NO in S11 or NO in S12, the operation time is shortened and the gas consumption amount is reduced.

In the control routine, S4 and S11 are equivalent to a "determination step" in the present invention, and the controller 11 of the gas supply target device 10 is equivalent to a "determination unit" of the present invention. Furthermore, a series of steps S5 to S10, a series of steps S12, S13, and S7 to S10, and a step of ending the control routine in case of NO in S11 are equivalent to "processing steps" in the present invention, and the controller 51 of the gas generator 50 is equivalent to a "processing unit" of the present invention.

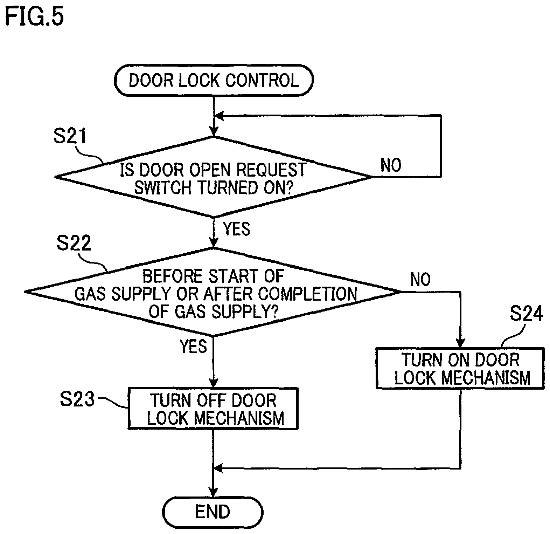

Now, the door lock control routine will be described with reference to FIG. 5. The control below is executed by the controller 11 of the gas supply target device 10 during the power supply to the gas supply target device 10.

To begin with, the controller 11 determines whether an ON signal has been sent from the door open request switch 16 (S21) . When the ON signal has not been sent from the door open request switch 16 (NO in S21), the controller 11 repeats the step S21.

When the ON signal has been sent from the door open request switch 16 (YES in S21), the controller 11 determines if the retaining unit 12 is in the state A indicating "before the start of the gas supply" or the state C indicating "after the completion of the gas supply" (S22) .

When the retaining unit 12 is in the state A indicating "before the start of the gas supply" or the state C indicating "after the completion of the gas supply" (YES in S22), the controller 11 switches off the door lock mechanism 17 after stopping portions of the gas supply target device 10 to be safe for operating personnel when the door lock mechanism 17 is in the on state at this stage, or the controller 11 maintains the door lock mechanism 17 in the off state when the door lock mechanism 17 is in the off state at this stage (S23). The control routine ends after S23.

When the retaining unit 12 is neither in the state A indicating "before the start of the gas supply" nor the state C indicating "after the completion of the gas supply" (i.e., is in the state B indicating "during the gas supply") (NO in S22), the controller 11 maintains the door lock mechanism 17 in the on state when the door lock mechanism 17 is in the on state at this stage, or switches on the door lock mechanism 17 when the door lock mechanism 17 is in the off state at this stage (S24) . The control routine ends after S24.

In the control routine, S22 is equivalent to the "determination step" in the present invention, and the controller 11 of the gas supply target device 10 is equivalent to the "determination unit" of the present invention. Furthermore, S23 and S24 are equivalent to a "processing step" of the present invention, and the controller 11 of the gas supply target device 10 is equivalent to a "processing unit" of the present invention.

As described above, according to the present embodiment, when the power supply to the gas supply target device 10 is stopped and then resumed, the state of the retaining unit 12 is determined (S4, S11, and S22) and the state of the gas supply to the chamber 10a (before the start of the gas supply, during the gas supply, or after the completion of the gas supply in the present embodiment) is grasped, and a process suitable for these states can be performed.

To be more specific, for example, instead of simply performing the aeration, the gas supply is continued (i.e., a series of steps S13 and S7 to S10 is executed) when the retaining unit 12 is in the state B indicating "during the gas supply" and the gas concentration is equal to or lower than a predetermined value (YES in S11 and then YES in S12) . This restrains the deterioration of the operation efficiency. Furthermore, for example, when the retaining unit 12 is in the state B indicating "during the gas supply" (NO in S22), the door 10c is prohibited from opening (S24). This assures the safety of the operating personnel.

It is noted that the gas supply to the gas supply target device 10 may be stopped not because of a power failure but because of the detachment of the intake pipe 73 and/or the exhaust pipe 74. Even in such a case, according to the present embodiment, the retaining unit is able to maintain one of the states A to C. On this account, at the stage of resuming the gas supply as the intake pipe 73 and/or the exhaust pipe 74 is attached again, in which one of the states A to C the retaining unit is in is determined (S4, S11, and S22), and a process suitable for the state of the gas supply to the chamber 10a is performed.

The retaining unit 12 is configured so that an external device (which is the controller 51 of the gas generator 50 in the present embodiment) be able to determine, by using the retaining unit 12, which one of the states A to C is set, both when power supply to the gas supply target device 10 is in process and when the power supply is stopped. According to this arrangement, both when power supply to the gas supply target device 10 is in process and when the power supply is stopped, the external device is able to grasp the state of the retaining unit 12 in the gas supply target device 10 and the state of the gas supply to the chamber 10a of the gas supply target device 10, and to perform a process suitable for these states.

When power supply to the gas supply target device 10 is in process, the retaining unit 12 can be set in one of the states A to C by an external device (which is the controller 51 of the gas generator 50 in the present embodiment) . According to this arrangement, the gas supply target device 10 is further suitably managed by the external device.

The retaining unit 12 includes the latching relays 12a and 12b. According to this arrangement, the state of the gas supply to the chamber 10a is accurately grasped by electrically determining the state of the latching relays 12a and 12b. It is therefore possible to perform a process suitable for the state.

The gas supplied to the chamber 10a has a sterilizing effect. The sterilizing effect indicates an effect to achieve complete elimination or removal of all kinds of proliferative microbiological organisms (mainly bacteria). Depending on the concentration, the gas having the sterilizing effect may be harmful for human body. According to the arrangement above, the advantage of the present invention is therefore significant in terms of the security of the operating personnel.

The gas supply system 1 includes a plurality of gas supply target devices 10 and is arranged to supply the gas generated by the gas generating unit 50a to the chamber 10a of one gas supply target device 10 which is selected one by one from the gas supply target devices 10. According to this arrangement, when the gas is supplied to the chambers 10a of the gas supply target devices 10 one by one, it is possible to grasp the state of the gas supply to each gas supply target device 10 and perform a process suitable for the state.

The retaining unit 12 is able to selectively take one of three or more states (three states A to C in the present embodiment). According to this arrangement, because the retaining unit 12 is able to selectively take not one of only two states but one of three or more states, it is possible to grasp the state of the gas supply to the chamber 10a in a detailed manner and to realize fine management of the gas supply target device 10.

The retaining unit 12 includes a plurality of elements (latching relays 12a and 12b), the state of each of which is switchable. According to this arrangement, the arrangement that the retaining unit is able to selectively take one of three or more states is relatively easily realized by combinations of the states of the elements.

A preferred embodiment of the present invention has been described.

It should be noted that the present invention is not limited to the above-described embodiment, and various changes, substitutions, and alterations can be made herein without departing from the spirit and scope of the invention as defined by the appended claims.

The shape of the container attached to the container attachment portion may be any shape, including SMIFPOD (Standard Mechanical Interface Pod: a container with a closable opening at a bottom portion) and FOUP (Front Opening Unified Pod: a container with a closable opening at a front surface) . The gas supply target device of the present invention is not limited to a part (e.g., the container attachment portion) where a cell culture dish is provided.

The gas supply target devices included in the gas supply system of the present invention may be structurally different from one another or structurally identical with one another. The gas supply system of the present invention may include only one gas supply target device.

The present invention is not limited to purposes such as cell culture and inspection of cells. The present invention is applicable to a gas supply target device to which gas is supplied and a system for supplying gas, which are provided for various purposes. Furthermore, the present invention is applicable to a device, a system, or the like for processing substrates such as semiconductor wafers and liquid crystal display panels.

The gas supplied to the chamber of the gas supply target device is not limited to hydrogen peroxide gas. The gas may be any type of gas such as ethylene oxide gas, nitrogen gas, argon gas, and air, or may be a combination of a plurality of types of gases. Furthermore, the gas is not limited to those having a sterilizing effect and harmful for human body.

The states of the gas supply to a chamber, which are states of the retaining unit, are not limited to "before the start of the gas supply", "during the gas supply", and "after completion of the gas supply" as in the embodiment above, and may be "gas concentration in the chamber", "the number of times of the gas supply to the chamber (history)", or the like.

The retaining unit is not limited to the latching relays. For example, the retaining unit may be a latching solenoid or a non-volatile memory (such as EPROM) . When the retaining unit is a latching solenoid, the state of the latching solenoid (first state or second state) is visually checkable. When the retaining unit is a latching solenoid, it is possible to prevent the door 10c from opening or prevent the pipes 73 and 74 from being detached, by associating the door lock mechanism 17, a lock mechanism for the pipes 73 and 74, and the like with the latching solenoid so as to cause the lock mechanism to be switched on when the latching solenoid is in the second state. When the retaining unit is a non-volatile memory, "gas concentration in the chamber" and/or "the number of times of the gas supply to the chamber" may be stored in the non-volatile memory.

The number of states that the retaining unit can take is more than one, and may be, for example, two ("first state" indicating "before the start of the gas supply" or "after the completion of the gas supply" and "second state" indicating "during the gas supply"). The number of elements included in the retaining unit may be three or more. Alternatively, the retaining unit may be formed of a single element (e.g., one latching relay).

The determination unit and the processing unit are provided inside or outside the gas supply target device.

The processes executed by the processing unit (i.e., processes executed in the processing step) are not limited to the processes described in the embodiment above (e.g., the process of prohibiting the door from opening), and may be a process of prohibiting an intake pipe and an exhaust pipe from being detached or a process of performing notification (e.g., by alarming sound or by an image) when the door is opened or when the intake pipe and the exhaust pipe are detached.

### [Reference Signs List]

1 gas supply system
10 gas supply target device
10a chamber
11 controller (determination unit, processing unit)
12 retaining unit
12a, 12b latching relay (element)
50 gas generator
50a gas generating unit
51 controller (processing unit)

## Claims

1. A gas supply target device comprising a chamber to which gas is supplied, the gas supply target device further comprising a retaining unit which is able to selectively take one of a plurality of states each indicating a state of gas supply to the chamber and is able to maintain, when power supply to the gas supply target device is stopped, one of the states which is set at stop of the power supply.

2. The gas supply target device according to claim 1, wherein, the retaining unit is configured so that an external device be able to determine, by using the retaining unit, which one of the states is set, both when the power supply to the gas supply target device is in process and when the power supply is stopped.

3. The gas supply target device according to claim 1 or 2, wherein, when the power supply to the gas supply target device is in process, the retaining unit is settable in one of the states by an external device.

4. The gas supply target device according to any one of claims 1 to 3, wherein, the retaining unit includes a latching relay.

5. The gas supply target device according to any one of claims 1 to 4, wherein, the gas has a sterilizing effect.

6. A gas supply system comprising:
a gas supply target device including a chamber to which gas is supplied; and
a gas generator including a gas generating unit configured to generate the gas,
the gas generated by the gas generating unit being supplied to the chamber,
the gas supply target device further including a retaining unit which is able to selectively take one of a plurality of states each indicating a state of gas supply to the chamber and is able to maintain, when power supply to the gas supply target device is stopped, one of the states at stop of the power supply, and
the gas supply system further comprising: a determination unit configured to determine in which one of the states the retaining unit is; and
a processing unit configured to perform a process in accordance with determination by the determination unit.

7. The gas supply system according to claim 4, further comprising at least one gas supply target device each of which is identical with the gas supply target device,
the gas generated by the gas generating unit being supplied to the chamber of one gas supply target device which is selected one by one from the gas supply target devices.

8. A method of controlling a gas supply system which comprises a gas supply target device including a chamber to which gas is supplied and a gas generator including a gas generating unit configured to generate the gas, the gas supply system being configured to supply the gas generated by the gas generator to the chamber,
the gas supply target device further including a retaining unit which is able to selectively take one of a plurality of states each indicating a state of gas supply to the chamber and is able to maintain, when power supply to the gas supply target device is stopped, one of the states at stop of the power supply, and the method comprising the step of: determining, by a determination unit of the gas supply system, in which one of the states the retaining unit is; and
performing, by a processing unit of the gas supply system, a process in accordance with determination of the determination unit.
